# EUROPEAN PATENT APPLICATION

(11) **EP 4 353 208 A1**
(43) Date of publication of application: **17.04.2024**
(21) Application number: 22382956.5
(22) Date of filing: 10.10.2022
(51) Int. Cl.: A61F 13/472, A61F 13/511, B32B 33/00, B32B 27/32, A61F 13/514

(54) **FEMININE HYGIENE ARTICLES AND FILM WITH IMPROVED SOFTNESS AND HAND FEEL**

(71) Applicant: Dow Global Technologies LLC, Midland, MI 48674 (US); Dow Quimica de Colombia S.A., Bogotá D.C., Colombia, C.P. 111111 (CO)
(72) Inventor: BRUNETTO, Michel, Jundiaí (BR); CARDONA JIMENEZ, Elkin David, 110221 Bogotá (CO); COLOMBO, Veronica, 43006 Tarragona (ES)
(74) Representative: Elzaburu S.L.P.

(57) **Abstract**

A feminine hygiene article comprising a film having at least a first layer and an optional second layer, the first layer comprising at least 25 wt.%, based on the total weight of the first layer, of an ethylene acrylate copolymer and less than 75 wt.%, based on the total weight of the first layer, of a polyolefin is disclosed. The first layer may have a gloss measured by ASTM D2457 at 45°C below 30% and a drapeability measured according to WSP 90.3 less than or equal to 10.0 gf in both the machine or cross direction. The ethylene acrylate copolymer may contain from 16-35 wt.%, based on the total weight of the ethylene acrylate copolymer, acrylate. A feminine hygiene article comprising a soft film of non plastic appearance but with high tensile strength, low elongation along with high tear and puncture resistance, and high modulus can thus be obtained.

## Description

### TECHNICAL FIELD

The current disclosure generally relates to feminine hygiene articles. More specifically the current disclosure relates to feminine hygiene articles comprising polyolefin films containing ethylene acrylate copolymers.

### INTRODUCTION

Feminine hygiene articles contact one of the most sensitive parts of human anatomy. Thus, the feminine hygiene market values items with a cotton or silk like feel and appearance despite the fact they are produced using polyethylene or polypropylene. Feminine and other hygiene articles must also absorb and retain liquid during everyday activities. Thus, high tensile strength, low elongation, high puncture and tear resistance, along with a high modulus are also important. These ensure the strength, stability, and suitability for printing of the final manufactured article as well.

Feminine hygiene articles can comprise a three layer or a four layer pad. A three layer pad can comprise a permeable top sheet, an absorbent core, and an impermeable back sheet. A four layer pad can comprise a permeable top sheet, a transfer layer, an absorbent core, and an impermeable back sheet.

Softness can be improved by reducing the thickness of the film coating the absorbent core of the hygiene article. Softness can also be improved by lowering the density of polyethylene used. This however dramatically affects mechanical strength and greatly increases elongation which makes printing and article assembly difficult.

Reduced gloss and increased opacity leading to a non-plastic appearance are often achieved through the addition of pigments and online or offline embossing. However, this is often difficult especially in perforated films such as ADLs or topsheets.

Thus, there is an ongoing need for feminine hygiene articles comprising soft films of non plastic appearance with high tensile strength, low elongation, along with high tear and puncture resistance, and high modulus.

### SUMMARY OF DISCLOSURE

A feminine hygiene article comprising a film having at least a first layer and an optional second layer, the first layer comprising at least 25 wt.%, based on the total weight of the first layer, of an ethylene acrylate copolymer and less than 75 wt.%, based on the total weight of the first layer, of a polyolefin is disclosed.

### BRIEF DESCRIPTION OF THE DRAWINGS

Figure 1 graphicly depicts the drapability, measured using a handle-o-meter according to WSP 90.3, of the inventive and comparative examples in both the machine and cross direction.
Figure 2 graphicly depicts both the average dynamic and average static coefficients of friction, measured according to ASTM 1894, of the inventive and comparative examples.
Figure 3 graphically depicts the gloss at 45°, measured according to ASTM D2457, of both the inventive and comparative examples.
Figure 4 contains magnified pictures of reflective light at the film surface of both the inventive and comparative examples.
Figure 5 graphically represents the tensile strength at break in both the machine and cross direction, measured according to ASTM D882, of the inventive and comparative examples.
Figure 6 graphically represents the elongation at break in both the machine and cross direction, measured according to ASTM D882, of the inventive and comparative examples.
Figure 7 graphically represents Elmendorf tear in both the machine and cross direction, measured according to ASTM D1922, of both the inventive and comparative examples.
Figure 8 graphically represents the puncture resistance, measured according to ASTM D5748, of the inventive and comparative examples.

### DETAILED DESCRIPTION

A film having at least a first layer comprising at least 25 wt.% of an ethylene acrylate copolymer will now be disclosed in detail in the following description and examples. A feminine hygiene article comprising the disclosed film having at least a first layer comprising at least 25 wt. % of an ethylene acrylate copolymer is also disclosed. A feminine hygiene article comprising the disclosed film having at least a first layer comprising at least 25 wt.% of an ethylene acrylate copolymer and a nonwoven is also disclosed.

The term "polymer" refers to a polymeric compound prepared by polymerizing monomers, whether of the same or a different type. The generic term polymer thus embraces the term "homopolymer," usually employed to refer to polymers prepared from only one type of monomer as well as "copolymer" which refers to polymers prepared from two or more different monomers. The term "interpolymer," as used herein, refers to a polymer prepared by the polymerization of at least two different types of monomers. The generic term interpolymer thus includes copolymers, and polymers prepared from more than two different types of monomers, such as terpolymers.

As used herein, a "polyolefin" refers to an olefin-based polymer. As used herein, an "olefin," which may also be referred to as an "alkene," refers to a linear, branched, or cyclic compound including carbon and hydrogen and having at least one double bond. As used herein, when a polymer or copolymer, e.g., the polyolefin elastomer, is referred to as comprising an olefin, the olefin present in the polymer or copolymer is the polymerized form of the olefin. For example, if the polyolefin elastomer is said to have an ethylene content of 75 wt% to 85 wt%, it is understood that the polymer unit in the polyolefin elastomer is derived from ethylene in the polymerization reaction and the derived units are present at 75 wt% to 85 wt%, based on the total weight of the polyolefin elastomer.

As used herein, the term "polyethylene" refers to polymers comprising greater than 50% by weight of units which are derived from ethylene monomer, and optionally, one or more comonomers. This may include polyethylene homopolymers or copolymers (meaning units derived from two or more comonomers). Common forms of polyethylene known in the art include Low Density Polyethylene (LDPE); Linear Low Density Polyethylene (LLDPE); Ultra Low Density Polyethylene (ULDPE); Very Low Density Polyethylene (VLDPE); single-site catalyzed Linear Low Density Polyethylene, including both linear and substantially linear low density resins (m-LLDPE); Medium Density Polyethylene (MDPE); and High Density Polyethylene (HDPE).

As used herein, the term "ethylene acrylate copolymer" refers to a copolymer that comprises, in polymerized form, a majority amount of ethylene monomer (based on the weight of the copolymer), and an alkyl acrylate.

As used herein, the term "meltblown" refers to the fabrication of nonwoven fabrics via a process which generally includes the following steps: (a) extruding molten thermoplastic strands from a spinneret; (b) simultaneously quenching and attenuating the polymer stream immediately below the spinneret using streams of high velocity heated air; (c) collecting the drawn strands into a web on a collecting surface. Meltblown webs can be bonded by a variety of means including, but not limited to, autogeneous bonding, i.e., self bonding without further treatment, thermo-calendaring process, adhesive bonding process, hot air bonding process, needle punch process, hydroentangling process, and combinations thereof.

As used herein, the term "spunbond" refers to the fabrication of nonwoven fabric including the following steps: (a) extruding molten thermoplastic strands from a plurality of fine capillaries called a spinneret; (b) quenching the strands with a flow of air which is generally cooled in order to hasten the solidification of the molten strands; (c) attenuating the stands by advancing them through the quench zone with a draw tension that can be applied by either pneumatically entraining the stands in an air stream or by winding them around mechanical draw rolls of the type commonly used in the textile fibers industry; (d) collecting the drawn strands into a web on a foraminous surface, e.g., moving screen or porous belt; and (e) bonding the web of loose strands into a nonwoven fabric. Bonding can be achieved by a variety of means including, but not limited to, thermo-calendaring process, adhesive bonding process, hot air bonding process, needle punch process, hydroentangling process, and combinations thereof.

As used herein, the terms "nonwoven," "nonwoven web," and "nonwoven fabric" are used herein interchangeably. "Nonwoven" refers to a web or fabric having a structure of individual fibers or threads which are randomly interlaid, but not in an identifiable manner as is the case for a knitted fabric.

### Film Composition and Properties

The disclosed first layer of the film may comprise at least 25 wt.%, based on the total weight of the first layer, ethylene acrylate copolymer. Ethylene acrylate copolymer includes but is not limited to ethylene methyl acrylate copolymer, an ethylene ethyl acrylate copolymer, or an ethylene n-butyl acrylate copolymer. For example, the first layer may comprise at least 25 wt.% of an ethylene methyl acrylate copolymer, an ethylene ethyl acrylate copolymer, or an ethylene n-butyl acrylate copolymer. The first layer may comprise at least 50 wt.%, based on the total weight of the first layer, ethylene acrylate copolymer. The first layer may comprise less than or equal to 75 wt.%, based on the total weight of the first layer, ethylene acrylate copolymer. The first layer may comprise from 25 to 75 wt.%, based on the total weight of the first layer, ethylene acrylate copolymer. All individual values and subranges are disclosed. For example, the first layer may comprise from 35 to 65 wt.%, based on the total weight of the first layer, ethylene acrylate copolymer. The first layer may comprise from 45 to 55 wt.%, based on the total weight of the first layer, ethylene acrylate copolymer.

The disclosed first layer may comprise less than 75 wt.%, based on the total weight of the first layer, of a polyolefin. The first layer may comprise less than 50%, based on the total weight of the first layer, of a polyolefin. The first layer may comprise less than 25%, based on the total weight of the first layer, of a polyolefin. The first layer may comprise from 25 to 75 wt.%, based on the total weight of the first layer, of a polyolefin. All individual values and subranges are disclosed. For example, the first layer may comprise from a lower limit of 25, 35, 45, 55, 65, or 70 wt.% to an upper limit of 75, 70, 65, 55, 45, 35, or 30 wt.% based on the total weight of the first layer, of a polyolefin.

The first layer may further comprise white pigment. The first layer may comprise at least 5 wt.% white pigment, based on the total weight of the first layer. The first layer may comprise at least 5 to 20 wt.% white pigment based on the total weight of the first layer. All individual values and subranges from 5 to 20 wt.% are included and disclosed. For example, the first layer may comprise 10 to 20 wt.% white pigment based on the total weight of the first layer. The first layer may comprise 15 to 20 wt.% white pigment based on the total weight of the first layer.

The first layer may further comprise calcium carbonate. The first layer may comprise at least 5 wt.% calcium carbonate based on the total weight of the first layer. The first layer may comprise 5 to 15 wt.% calcium carbonate based on the total weight of the first layer. All individual values and subranges from 5 to 15 wt.% are included and disclosed. For example, the first layer may comprise 5 to 10 wt.% calcium carbonate.

The first layer may have a gloss measured by ASTM D2457 at 45° below 40%. The first layer may have a gloss measured by ASTM D2457 at 45° below 30%. The first layer may have a gloss measured by ASTM D2457 at 45° below 20%. The first layer may have a gloss measured by ASTM D2457 at 45° between 10 to 40%. All individual values and subranges are disclosed. For example, the first layer may have a gloss measured by ASTM D2457 at 45° from 20 to 30%. The first layer may have a gloss measured by ASTM D2457 at 45° from 10 to 20%.

The film may have a drapeability measured according to WSP 90.3 less than or equal to 10.0 gf in both the machine and the cross direction. The first layer may have a drapeability measured according to WSP 90.3 less than or equal to 5.0 gf in both the machine and the cross direction. The first layer may have a drapeability measured according to WSP 90.3 less than or equal to 4.0 gf in both the machine and the cross direction. The first layer may have a drapeability measured according to WSP 90.3 less than or equal to 3.0 gf in both the machine and the cross direction. The first layer may have a drapeability measured according to WSP 90.3 from 2.0 to 10.0 gf in both the machine and cross direction. All individual values and subranges are included and disclosed. For example, the first layer may have a drapeability measured according to WSP 90.3 from a lower limit of 2.0, 2.5, 3.0, 3.5, 4.0, 4.5 or 5.0 gf to an upper limit of 10.0, 9.5, 9.0, 8.5, 8.0, 7.5, 7.0, 6.5, 6.0, 5.5, 5.0, 4.5, 4.0, or 3.5 gf, in both the machine and the cross direction.

The disclosed film may further comprise a second and a third layer. The second layer and the third layer may each comprise separate polyethylene compositions.

### Polymer Composition and Production

Suitable acrylates for use in the first layer of the film include, but are not limited to, methyl acrylate, ethyl acrylate, and n-butyl acrylate. The ethylene acrylate copolymer may comprise at least 16 wt.% acrylate. The ethylene acrylate copolymer may comprise from 16-35 wt.%, based on the total weight of the ethylene acrylate copolymer, acrylate. All individual values and subranges from 16-35 wt.% are included and disclosed. For example, the ethylene acrylate copolymer, may comprise from 20-35 wt.%, based on the total weight of the ethylene acrylate copolymer, acrylate. Examples of commercially available ethylene acrylate resins suitable for use in this disclosure include ELVALOY^{™} AC 1224 or 1330 available from The Dow Chemical Company (Midland, MI).

The polyolefin of the first layer may comprise an elastomer. Such a polyolefin elastomer may comprise an olefin block copolymer, for example, an ethylene/alpha-olefin block copolymer. Commercial examples of ethylene/alpha-olefin block copolymer include resins available under the trade name INFUSE^{™}, available from The Dow Chemical Company (Midland, MI).

The ethylene/alpha-olefin block copolymer may comprise ethylene and one or more copolymerizable alpha-olefins in polymerized form, characterized by multiple blocks or segments of two or more polymerized monomer units differing in chemical or physical properties. The polyolefin elastomers can be multi-block copolymers. The multi-block copolymer can be represented by the formula (AB)n where n is at least 1, preferably an integer greater than 1, such as 2, 3, 4, 5, 10, 15, 20, 30, 40, 50, 60, 70, 80, 90, 100, or higher, "A" represents a hard block, and "B" represents a soft block. As and Bs can be linked in a substantially linear fashion, as opposed to a substantially branched or substantially star-shaped fashion. The A blocks and B blocks can be randomly distributed along the polymer chain. Each of block A and block B can have monomers or comonomers substantially randomly distributed within the block, such that neither block A nor block B comprises two or more sub-segments of distinct composition, such as a tip segment, that has a substantially different composition than the rest of the block.

The ethylene/alpha-olefin block copolymer can include various amounts of "hard" and "soft" blocks. "Hard" blocks refer to blocks of polymerized units in which ethylene is present in an amount greater than about 95 wt%, and preferably greater than about 98 wt%, based on the weight of the polymer. In other words, the comonomer content, i.e., the content of monomers other than ethylene, in the hard blocks is less than about 5 wt%, and preferably less than about 2 wt%, based on the weight of the polymer. The hard blocks can comprise all or substantially all ethylene. "Soft" blocks, on the other hand, refer to blocks of polymerized units in which the comonomer content, i.e., the content of monomers other than ethylene, is greater than about 5 wt%. For example, the comonomer content may be greater than about 5 wt%, greater than about 8 wt%, greater than about 10 wt%, or greater than about 15 wt%, based on the weight of the polymer. The comonomer content in the soft blocks can be greater than about 20 wt%, greater than about 25 wt%, greater than about 30 wt%, greater than about 35 wt%, greater than about 40 wt%, greater than about 45 wt%, greater than about 50 wt%, or greater than about 60 wt%, based on the weight of the polymer.

The soft blocks can be present in the ethylene/alpha-olefin block copolymer at amounts from about 1 wt% to about 99 wt% of a total weight of the polyolefin elastomer. For example, the soft blocks can be present in the ethylene/alpha-olefin block copolymer from about 5 wt% to about 95 wt%, from about 10 wt% to about 90 wt%, from about 15 wt% to about 85 wt%, from about 20 wt% to about 80 wt%, from about 25 wt% to about 75 wt%, from about 30 wt% to about 70 wt%, from about 35 wt% to about 65 wt%, from about 40 wt% to about 60 wt%, or from about 45 wt% to about 55 wt% of the total weight of the polyolefin elastomer. Conversely, the hard blocks can be present in similar ranges. The soft block and hard block weight percentages can be calculated based on data obtained from DSC or NMR. Such methods and calculations are disclosed in U.S. Patent Application Publication No. 2006/0199930.

Examples of alpha-olefin comonomers that may be used to form the ethylene/alpha-olefin block copolymer include, by way of example and not limitation, propylene, isobutylene, 1-butene, 1-pentene, 1-hexene, 1-heptene, 1-octene, 1-nonene, 1-decene, and 1-dodecene, 1-tetradecene, 1-hexadecene, 1-octadecent, 1-eicosene, 3-methyl-1-butene, 3-methyl-1-penten, 4-methyl-1-penten, 4,6-dimethyle-1-heptene, 4-vinylcyclohexene, vinylcyclohexane, norbornadiene, ethylidene norbornene, cyclopentene, cyclohexene, dicyclopentadiene, cyclooctene, C₄-C₄₀ dienes, other C₄-C₄₀ alpha-olefins, and the like. The alpha-olefin comonomers may comprise C₃-C₈ comonomers. Comonomer content may be measured using any suitable technique, such as techniques based on nuclear magnetic resonance ("NMR") spectroscopy, and, for example, by ¹³C NMR analysis as described in U.S. Patent No. 7,498,282, which is incorporated herein by reference.

The first layer comprises a polyolefin. The polyolefin can be a polyethylene.

The polyethylene may have a density from 0.925 to 0.950 g/cm³. All individual values and subranges are included and disclosed. For example, the polyethylene may have a density from 0.930 to 0.945 g/cm³.

The polyethylene may have a melt index (12) from 0.50 to 10.00 g/10 min. All individual values and subranges are included and disclosed. For example, the polyethylene may have a melt index (12) from a lower limit of 0.50, 1.00, 2.00, 3.00, 4.00, 5.00, g/10 min to an upper limit of 9.00, 8.00, 7.00, 6.00, 5.00, 4.00, 3.00, or 2.00 g/10 min.

The polyethylene polymer suitable for use in the present disclosure may be commercially available. Suitable commercial polyethylene polymers include but are not limited to: AGILITY^{™} (e.g., AGILITY^{™} 1000, AGILITY^{™} 1001, and AGILITY ^{™}1021), INNATE^{™} ST 50, ELITE^{™} 5940, ELITE^{™} 5960, DOW^{™} LDPE 6211, and DOW^{™} LDPE 7511, all of which are available from The Dow Chemical Company.

Any conventional polymerization processes can be employed to produce the ethylene acrylate copolymer and the polyolefin. Such conventional polymerization processes include, but are not limited to, solution polymerization process, using one or more conventional reactors e.g. loop reactors, isothermal reactors, stirred tank reactors, batch reactors in parallel, series, and/or any combinations thereof. Such conventional polymerization processes also include gas-phase, solution or slurry polymerization or any combination thereof, using any type of reactor or reactor configuration known in the art.

The solution phase polymerization process can occur in one or more well-stirred reactors such as one or more loop reactors at a temperature in the range of from 115 to 250 °C; for example, from 155 to 225 °C, and at pressures in the range of from 300 to 1000 psi; for example, from 400 to 750 psi. In a dual reactor, the temperature in the first reactor temperature can be in the range of from 115 to 190 °C, for example, from 115 to 150 °C, and the second reactor temperature can be in the range of 150 to 200 °C, for example, from 170 to 195 °C. In a single reactor, the temperature in the reactor temperature can be in the range of from 115 to 250 °C, for example, from 155 to 225°C. The residence time in a solution phase polymerization process is typically in the range of from 2 to 30 minutes; for example, from 10 to 20 minutes. Ethylene, solvent, one or more catalyst systems, optionally one or more cocatalysts, optionally one or more impurity scavengers, and optionally one or more comonomers are fed continuously to one or more reactors. Exemplary solvents include, but are not limited to, isoparaffins. For example, such solvents are commercially available under the name ISOPAR E from ExxonMobil Chemical Co., Houston, Texas. The resultant mixture of polymer composition and solvent is then removed from the reactor and the polymer composition is isolated. Solvent is typically recovered via a solvent recovery unit, i.e. heat exchangers and vapor liquid separator drum, and is then recycled back into the polymerization system.

Polyolefin and ethylene acrylate copolymer may be produced via a solution polymerization process in a dual reactor system, for example a dual loop reactor system, wherein polyolefin, polyethylene, or ethylene acrylate copolymer are polymerized in the presence of one or more catalyst systems. Additionally, one or more co-catalysts may be present. Polyolefin and ethylene acrylate copolymer may be produced via a solution polymerization process in a single reactor system, for example a single loop reactor system, wherein polyolefin or ethylene acrylate copolymer are polymerized in the presence of one or more catalyst systems.

An example of a suitable catalyst system comprising a procatalyst component comprising a metal-ligand complex of formula (I):

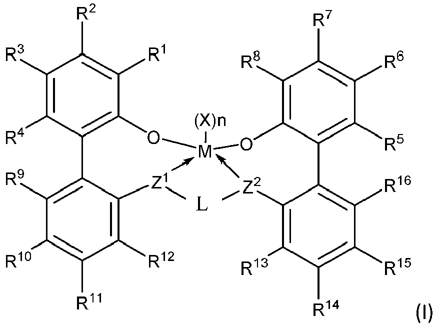

In formula (I), M is a metal chosen from titanium, zirconium, or hafnium, the metal being in a formal oxidation state of +2, +3, or +4; n is 0, 1, or 2; when n is 1, X is a monodentate ligand or a bidentate ligand; when n is 2, each X is a monodentate ligand and is the same or different; the metal-ligand complex is overall charge-neutral; each Z is independently chosen from -O-, -S-, -N(R^{N})-, or -P(R^{P})-, wherein independently each R^{N} and R^{P} is (C1-C30)hydrocarbyl or (C1-C30)heterohydrocarbyl; L is (C₁-C₄₀)hydrocarbylene or (C₁-C₄₀)heterohydrocarbylene, wherein the (C₁-C₄₀)hydrocarbylene has a portion that comprises a 1-carbon atom to 10-carbon atom linker backbone linking the two Z groups in Formula (I) (to which L is bonded) or the (C₁-C₄₀)heterohydrocarbylene has a portion that comprises a 1-atom to 10-atom linker backbone linking the two Z groups in Formula (I), wherein each of the 1 to 10 atoms of the 1-atom to 10-atom linker backbone of the (C₁-C₄₀)heterohydrocarbylene independently is a carbon atom or heteroatom, wherein each heteroatom independently is O, S, S(O), S(O)₂, Si(R^{C})₂, Ge(R^{C})₂, P(R^{C}), or N(R^{C}), wherein independently each R^{C} is (C₁-C₃₀)hydrocarbyl or (C₁-C₃₀)heterohydrocarbyl; R¹ and R⁸ are independently selected from the group consisting of - H, (C₁-C₄₀)hydrocarbyl, (C₁-C₄₀)heterohydrocarbyl, -Si(R^{C})₃, -Ge(R^{C})₃, -P(R^{P})₂, -N(R^{N})₂, -OR^{C}, -SR^{C}, -NO₂, -CN, -CF₃, R^{C}S(O)-, R^{C}S(O)₂-, (R^{C})₂C=N-, R^{C}C(O)O-, R^{C}OC(O)-, R^{C}C(O)N(R^{N})-, (R^{N})₂NC(O)-, halogen, and radicals having formula (II), formula (III), or formula (IV):

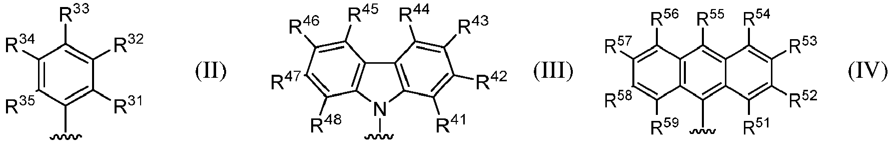

In formulas (II), (III), and (IV), each of R³¹⁻³⁵, R⁴¹⁻⁴⁸, or R⁵¹⁻⁵⁹ is independently chosen from (C₁-C₄₀)hydrocarbyl, (C₁-C₄₀)heterohydrocarbyl, -Si(R^{C})₃, -Ge(R^{C})₃, -P(R^{P})₂, -N(R^{N})₂, - N=CHR^{C}, -OR^{C}, -SR^{C}, -NO₂, -CN, -CF₃, R^{C}S(O)-, R^{C}S(O)₂-, (R^{C})₂C=N-, R^{C}C(O)O-, R^{C}OC(O)-, R^{C}C(O)N(R^{N})-, (R^{N})₂NC(O)-, halogen, or -H, provided at least one of R¹ or R⁸ is a radical having formula (II), formula (III), or formula (IV) where R^{C}, R^{N}, and R^{P} are as defined above.

In formula (I), each of R²⁻⁴, R⁵⁻⁷, and R⁹⁻¹⁶ is independently selected from (C₁-C₄₀)hydrocarbyl, (C₁-C₄₀)heterohydrocarbyl, -Si(R^{C})₃, -Ge(R^{C})₃, -P(R^{P})₂, -N(R^{N})₂, -N=CHR^{C}, -OR^{C}, -SR^{C}, -NO₂, -CN, -CF₃, R^{C}S(O)-, R^{C}S(O)₂-, (R^{C})₂C=N-, R^{C}C(O)O-, R^{C}OC(O)-, R^{C}C(O)N(R^{N})-, (R^{C})₂NC(O)-, halogen, and -H where R^{C}, R^{N}, and R^{P} are as defined above.

The catalyst system comprising a metal-ligand complex of formula (I) may be rendered catalytically active by any technique known in the art for activating metal-based catalysts of olefin polymerization reactions. For example, a metal-ligand complex of formula (I) may be rendered catalytically active by contacting the complex to, or combining the complex with, an activating co-catalyst. Suitable activating co-catalysts for use herein include alkyl aluminums; polymeric or oligomeric alumoxanes (also known as aluminoxanes); neutral Lewis acids; and non-polymeric, non-coordinating, ion-forming compounds (including the use of such compounds under oxidizing conditions). A suitable activating technique is bulk electrolysis. Combinations of one or more of the foregoing activating co-catalysts and techniques are also contemplated. The term "alkyl aluminum" means a monoalkyl aluminum dihydride or monoalkylaluminum dihalide, a dialkyl aluminum hydride or dialkyl aluminum halide, or a trialkylaluminum. Examples of polymeric or oligomeric alumoxanes include methylalumoxane, triisobutylaluminum-modified methylalumoxane, and isobutylalumoxane.

Lewis acid activators (co-catalysts) include Group 13 metal compounds containing from 1 to 3 (C₁-C₂₀)hydrocarbyl substituents as described herein. Examples of Group 13 metal compounds are tri((C₁-C₂₀)hydrocarbyl)-substituted-aluminum or tri((C₁-C₂₀)hydrocarbyl)-boron compounds; tri(hydrocarbyl)-substituted-aluminum, tri((C₁-C₂₀)hydrocarbyl)-boron compounds; tri((C₁-C₁₀)alkyl)aluminum, tri((C₆-C₁₈)aryl)boron compounds; and halogenated (including perhalogenated) derivatives thereof. In further examples, Group 13 metal compounds are tris(fluoro-substituted phenyl)boranes, tris(pentafluorophenyl)borane. An activating co-catalyst can be a tris((C₁-C₂₀)hydrocarbyl borate (e.g. trityl tetrafluoroborate) or a tri((C₁-C₂₀)hydrocarbyl)ammonium tetra((C₁-C₂₀)hydrocarbyl)borane (e.g. bis(octadecyl)methylammonium tetrakis(pentafluorophenyl)borane). As used herein, the term "ammonium" means a nitrogen cation that is a ((C₁-C₂₀)hydrocarbyl)₄N⁺, a ((C₁-C₂₀)hydrocarbyl)₃N(H)⁺, a ((C₁-C₂₀)hydrocarbyl)₂N(H)₂⁺, (C₁-C₂₀)hydrocarbylN(H)₃⁺, or N(H)₄⁺, wherein each (C₁-C₂₀)hydrocarbyl, when two or more are present, may be the same or different.

Combinations of neutral Lewis acid activators (co-catalysts) include mixtures comprising a combination of a tri((C₁^{∗}C₄)alkyl)aluminum and a halogenated tri((C₆^{∗}C₁₈)aryl)boron compound, especially a tris(pentafluorophenyl)borane; or combinations of such neutral Lewis acid mixtures with a polymeric or oligomeric alumoxane, and combinations of a single neutral Lewis acid, especially tris(pentafluorophenyl)borane with a polymeric or oligomeric alumoxane. Ratios of numbers of moles of (metal-ligand complex) : (tris(pentafluoro-phenylborane): (alumoxane) [e.g., (Group 4 metal-ligand complex) :(tris(pentafluoro-phenylborane):(alumoxane)] are from 1:1:1 to 1:10:30, or from 1:1:1.5 to 1:5:10.

The catalyst system comprising the metal-ligand complex of formula (I) can be activated to form an active catalyst composition by combination with one or more co-catalysts, for example, a cation forming co-catalyst, a strong Lewis acid, or combinations thereof. Suitable activating co-catalysts include polymeric or oligomeric aluminoxanes, especially methyl aluminoxane, as well as inert, compatible, noncoordinating, ion forming compounds. Exemplary suitable co-catalysts include, but are not limited to: modified methyl aluminoxane (MMAO), bis(hydrogenated tallow alkyl)methyl, tetrakis(pentafluorophenyl)borate(1⁻) amine, and combinations thereof.

One or more of the foregoing activating co-catalysts can be used in combination with each other. One preferred combination is a mixture of a tri((C₁-C₄)hydrocarbyl)aluminum, tri((C₁-C₄)hydrocarbyl)borane, or an ammonium borate with an oligomeric or polymeric alumoxane compound. The ratio of total number of moles of one or more metal-ligand complexes of formula (I) to total number of moles of one or more of the activating co-catalysts is from 1:10,000 to 100:1. The ratio can be at least 1:5000, or, at least 1:1000; and can be no more than 10:1 or no more than 1:1. When an alumoxane alone is used as the activating co-catalyst, preferably the number of moles of the alumoxane that are employed can be at least 100 times the number of moles of the metal-ligand complex of formula (I). When tris(pentafluorophenyl)borane alone is used as the activating co-catalyst, the ratio of the number of moles of the tris(pentafluorophenyl)borane that are employed to the total number of moles of one or more metal-ligand complexes of formula (I) can be from 0.5: 1 to 10:1, from 1:1 to 6:1, or from 1:1 to 5:1. The remaining activating co-catalysts are generally employed in approximately mole quantities equal to the total mole quantities of one or more metal-ligand complexes of formula (I).

### Film, Composite and Article Production

The disclosed film may be made by any process known in the art. These include, but are not limited to, cast machine direction orientation wherein the polymer is extruded through a flat die to create a flat solid film and then uniaxially-oriented in the machine direction at an elevated temperature, such as, from 20 °C to 50 °C lower than the melting point of the polymer. The films described herein may also be made using blown machine direction orientation whereby the polymer is extruded through an annular die and creates a tube of film that can be slit to create a solid flat film and then uniaxially-oriented in the machine direction at an elevated temperature, such as, from 20 °C to 50 °C lower than the melting point of the polymer.

A typical extrusion setup consists of having a hopper in the process upstream and the die in the process downstream. The hopper feeds the polymer into the barrel of the extruder, which contains the screw. The screw typically can be divided into 3 sections, i.e., the feed section, the compression section, and the metering section. Along the barrel of the extruder, there will be multiple heating zones from the rear to the front. The screw conveys the polymer forward while simultaneously melting and compressing the polymer melt inside the barrel of the extruder. The compression ratio of the screw is typically from 2.5 to 3.5. The length to diameter ratio of the barrel for such an extruder setup is 16:1 to 30:1. The extrusion process can occur at temperatures in the range of 160 to 270 degrees Celsius. At the end of the barrel, which is at the downstream end, between the screw and the die, is a breaker plate and screen pack.

Titanium dioxide and calcium carbonate may be added during film production as is known in the art.

Composites comprising the films as described herein laminated to a nonwoven are also disclosed. Feminine hygiene articles comprising the disclosed composites are disclosed. Feminine hygiene articles comprising the film as described herein are disclosed. A feminine hygiene article comprising a topsheet wherein the topsheet comprises the film described above is disclosed. For example, the feminine hygiene articles disclosed herein may comprise the film disclosed herein and further comprise a nonwoven. "Nonwoven" includes nonwoven webs, nonwoven fabrics, and any nonwoven structure in which individual fibers or threads are interlaid, but not in a regular or repeating manner. Nonwovens described herein may be formed by a variety of processes, such as, for example, air laying processes, meltblowing processes, spunbonding processes and carding processes, including bonded carded web processes.

### TEST METHODS

### Softness Evaluation

Drapeability of the film is evaluated using a handle-o-meter test according to the WSP 90.3 standard. The coefficient of friction is measured according to ASTM 1894 as a measure of hand feel cotton touch.

### Mechanical Properties Evaluation

The tensile strength and elongation is measured according to ASTM D882. Elmendorf Tear is measured according to ASTM D1922 while puncture was measured according to ASTM D5748.

### Gloss:

Gloss is measured at 45° deg according to ASTM D2457 and magnified pictures of reflective light are taken at the film surface.

### Density

Density is measured in accordance with ASTM D792 and expressed in grams/cm³ (g/cm³).

### Melt Index

Melt index (I2) is measured in accordance with ASTM D-1238 at 190°C at 2.16 kg. The values are reported in g/10 min; which corresponds to grams eluted per 10 minutes.

### EXAMPLES

Materials used in both the inventive and comparative examples are listed below in Table 1. All of the resins listed below are commercially available from the Dow Chemical Company.

**Table 1: Materials**

| Material | Density (g/cm³) | Melt index (I2) (g/10min) |
|---|---|---|
| AGILITY^{™} 6047 Performance LDPE | 0.947 | 6.00 |
| ELITE^{™} 5230 G Enhanced Polyethylene Resin | 0.916 | 4.00 |
| INFUSE^{™} 9507 Olefin block copolymer | 0.866 | 5.00 |
| AGILITY^{™} EC 7000 Performance LDPE | 0.918 | 3.90 |
| ELVAX^{™} 260 Ethylene Vinyl Acetate Copolymer | 0.955 | 2.00 |
| AFFINITY^{™} VP 8770G1 Polyolefin Plastomer | 0.887 | 1.00 |
| ELVALOY^{™} AC1330 Acrylate Copolymer | 0.950 | 3.00 |
| White Pigment (masterbatch 70% TiO₂) | | |
| Calcium Carbonate (masterbatch 70% CaCO₃) | | |

Co-extrusion of multilayer films is well known in the art and includes co-extrusion blown films and cast films as described, for example, in U.S. Pat. Nos. 3,479,425, 3,797,987, 3,959,431, and 4,406,547. Multilayered films can be produced by co-extruding two or more melt streams through a die to produce a layered structure when allowed to cool. The inventive and comparative films are produced in a Collin Cast pilot line with 600 mm width and 3 co-extruded layers in the following configuration: A-B-C, where the A is the bottom layer and C is the top layer. The die gap is 0.7 mm, extrusion temperature set on 270°C and die temperature set on 280°C. The production rate is set in 10 kg/hour where layer A corresponds to 35%, layer B to 45% and layer C to 20% of the whole thickness. The line speed is set to 11.88 m/min to produce a film with a final thickness of 25 microns or approximately 23 gsm (grams per square meter). Table 2 below shows the formulations of the first layer / top layer, layer C. The percentages in Table 2 are the weight percent (wt.%) based on the total weight of the first layer. In addition to the materials in Table 2, the first layer of the reference along with the inventive and comparative examples also comprises 15 wt.% white pigment.

**Table 2: Experimental and Comparative Formulations for First Layer with 15% White Pigment**

| | AGILITY^{™} 6047 | INFUSE^{™} 9507 | AGILITY^{™} EC 7000 | EVA^{™} 260 | Calcium Carbonate | AFFINTY^{™} VP8770 | ELVALOY^{™} AC 1330 |
|---|---|---|---|---|---|---|---|
| Reference | 50% | | 35% | | | | |
| IE1 | | | 35% | | | | 50% |
| IE2 | | | 25% | | 10% | | 50% |
| IE3 | 25% | | | | 10% | | 50% |
| CE1 | | 50% | 35% | | | | |
| CE2 | | | 35% | | | 50% | |
| CE3 | | | 35% | 50% | | | |

Each sample above is a three layer film with the first layer as an outer layer / top layer. This layer comprises 20% of each structure. The other outer layer / bottom layer comprises 35% of each structure and comprises 60% AGILITY^{™} 6047, and 40% AGILITY^{™} EC 7000. The core / middle layer comprises 45% of each structure and comprises 50% AGILITY^{™} 6047, 20% ELITE^{™} 5230G and 30% AGILITY^{™} EC 7000. The composition of the bottom and middle layers can vary according to the final application and product requirements.

**Table 3: Properties of Inventive and Comparative Examples**

| | Reference | IE-1 | IE-2 | IE-3 | CE-1 | CE-2 | CE-3 |
|---|---|---|---|---|---|---|---|
| Drapeability (gf) MD | 5.7 | 2.8 | 2.7 | 3.2 | 3.3 | 3.8 | 3.5 |
| Drapeability (gf) CD | 6.9 | 3.4 | 3.2 | 3.5 | 4.1 | 3.7 | 3.3 |
| Coefficient of Friction Average Dynamic | 0.33 | 1.63 | 1.35 | 1.20 | 2.37 | 1.23 | 1.27 |
| Coefficient of Friction Average Static | 0.35 | 1.49 | 1.24 | 1.18 | 1.58 | 1.11 | 1.13 |
| Gloss at 45° (%) | 46 | 24 | 16 | 16 | 40 | 44 | 40 |
| Tensile Strength at Break (N/5cm) MD | 21.7 | 20.7 | 21.4 | 18.3 | 15.5 | 22.7 | 19.8 |
| Tensile Strength at Break (N/5cm) CD | 16.3 | 18.1 | 18.7 | 14.3 | 16.4 | 21.1 | 19.2 |
| Elongation at Break (%) MD | 524 | 544 | 620 | 559 | 465 | 636 | 550 |
| Elongation at Break (%) CD | 707 | 766 | 748 | 704 | 772 | 801 | 778 |
| Elmendorf Tear (gf) MD | 26 | 122 | 69 | 57 | 79 | 102 | 72 |
| Elmendorf Tear (gf) CD | 326 | 325 | 316 | 352 | 322 | 346 | 322 |
| Avg-Puncture Resistance (J/cm³) | 1.54 | 1.61 | 0.93 | 1.14 | 2.39 | 2.28 | 1.46 |

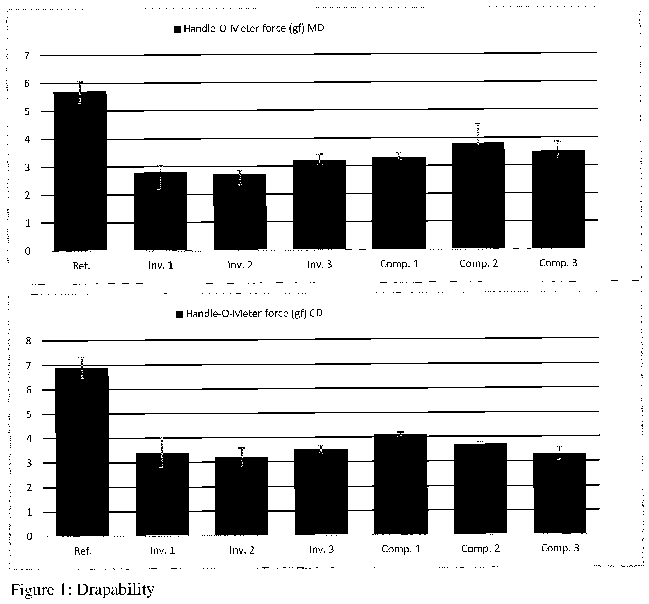

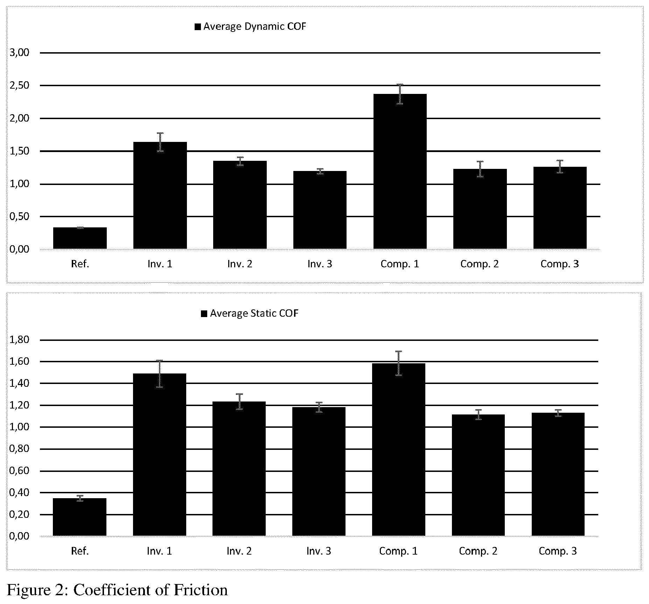

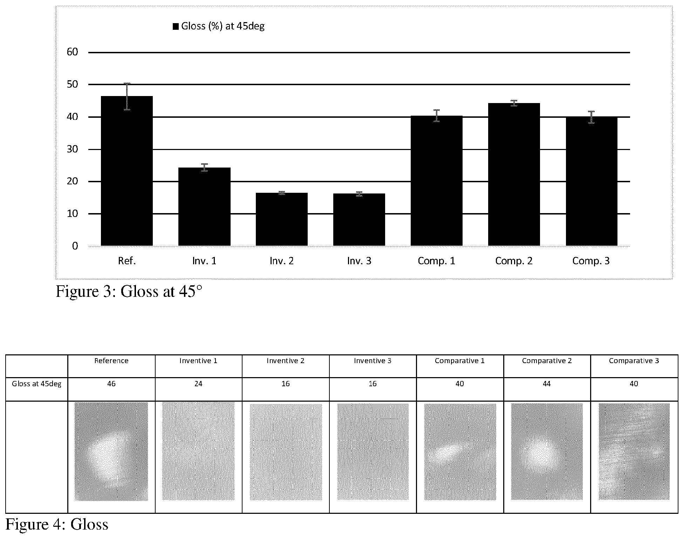

As can be seen in figure 1, inventive examples 1, 2 and 3 (IE-1, IE-2, and IE-3) have a drapeability two times that of the reference, and consistently better than the comparative samples. As can be seen in figure 2, the inventive examples have a higher coefficient of friction than the reference and compares favorably to all the comparative examples except CE-1. CE-1, however, is undesirably rubbery and sticky. Figures 3 and 4 show the significant improvement in opacity demonstrated by the numerical measurements of gloss along with the amplified photos of the film surface showing luminosity. Finally, a seven person human sensory panel observed higher softness and less noise generation upon sample handling of IE-3 when compared to the reference in table 2.

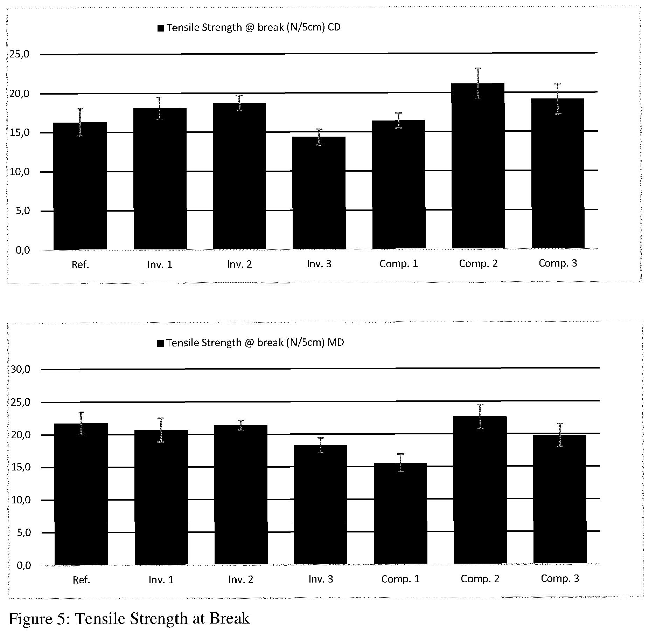

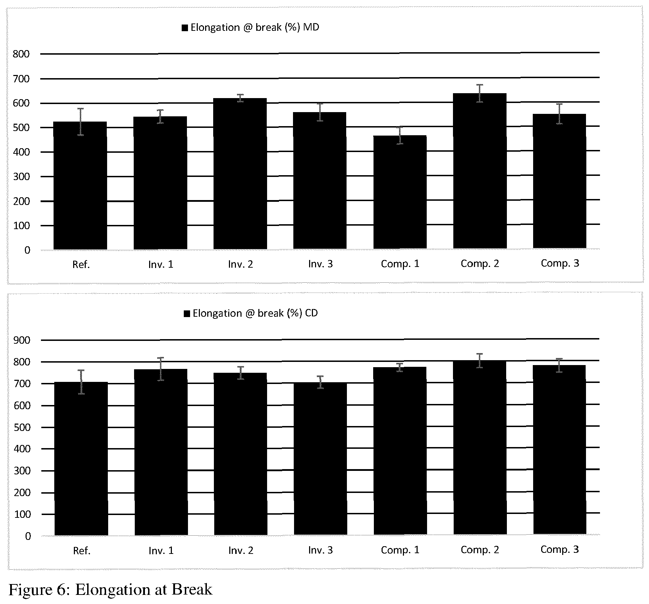

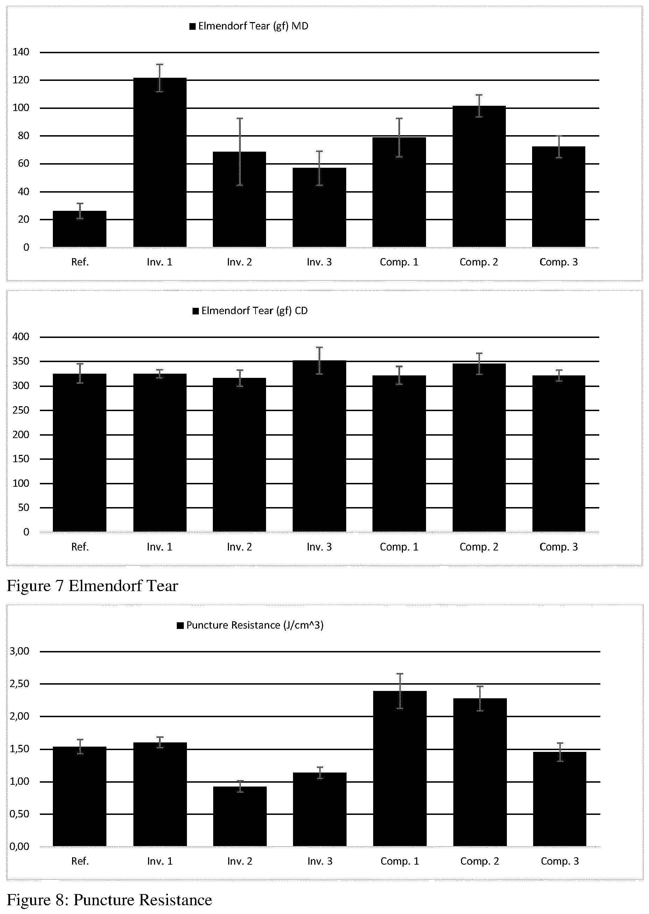

All of this is achieved without using lower density or lower crystallinity resins. As can be seen in figures 5-7, this is also achieved without sacrificing the mechanical properties of the film. In fact, as can be seen in figure 5, the machine direction tear strength is actually improved in IE-1 and IE-2 when compared to the reference.

## Claims

1. A feminine hygiene article comprising a film having at least a first layer and an optional second layer, the first layer comprising at least 25 wt.%, based on the total weight of the first layer, of an ethylene acrylate copolymer and less than 75 wt.%, based on the total weight of the first layer, of a polyolefin.

2. The feminine hygiene article of claim 1, wherein the ethylene acrylate copolymer is an ethylene methyl acrylate copolymer, an ethylene ethyl acrylate copolymer, or an ethylene n-butyl acrylate copolymer.

3. The feminine hygiene article of claims 1 or 2, wherein the ethylene acrylate copolymer comprises at least 16 wt.% acrylate, based on the total weight of the ethylene acrylate copolymer.

4. The feminine hygiene article of any of the preceding claims, wherein the first layer further comprises white pigment.

5. The feminine hygiene article of any of the preceding claims, wherein the polyolefin is a polyethylene having a density of 0.925 to 0.950 g/cm³ and a melt index (I2) of 0.50 to 10.00 g/10 min.

6. The feminine hygiene article of any of the preceding claims, wherein the first layer further comprises calcium carbonate.

7. The feminine hygiene article of any of the preceding claims, wherein the first layer comprises at least 5 wt.% white pigment, based on the total weight of the first layer.

8. The feminine hygiene article of any of the preceding claims, wherein the film further comprises a second layer and a third layer, the second layer and third layer each separately comprising a polyethylene composition.

9. The feminine hygiene article of any of the preceding claims, wherein the first layer has a gloss of below 40%.

10. The feminine hygiene article of any of the preceding claims, wherein the film has a drapability of less than 10.0 gf in both the machine and cross direction.

11. The feminine hygiene article of any of the preceding claims, further comprising a nonwoven.

12. A feminine hygiene article comprising a pad having a topsheet wherein the topsheet comprises the film of claim 1.
